# EUROPEAN PATENT SPECIFICATION

(11) **EP 2 853 587 B1**
(45) Date of publication and mention of the grant of the patent: **11.10.2017**
(21) Application number: 14003562.7
(22) Date of filing: 31.05.2010
(51) Int. Cl.: C12N 1/02, C12Q 1/02, C12Q 1/24, C12N 1/20, C12N 5/00

(54) **Method for isolating cells**
Verfahren zur Isolierung von Zellen
Procédé pour isoler des cellules

(30) Priority: 18.06.2009 EP 09007959
(43) Date of publication of application: 01.04.2015
(62) Divisional of application: 10723931.1
(73) Proprietor: Merck Patent GmbH, 64293 Darmstadt (DE)
(72) Inventor: Rossmanith, Peter, 2531 Gaaden (AT); Mester, Patrick Julian, 1020 Wien (AT); Wagner, Martin, 1180 Wien (AT)

(56) References cited:
- EP-A1- 1 882 738
- WO-A1-2008/017097
- WO-A2-2006/069413
- US-A- 5 700 645
- DATABASE WPI Week 200508 Thomson Scientific, London, GB; AN 2005-067100 XP002736178, -& CN 1 529 164 A (UNIV NANJING AGRIC) 15 September 2004 (2004-09-15)
- Johnson Jennifer L.: "Isolation & Identification of pathogenic Yersinia enterocolitica from meat and poultry products", USDA/FSIS Microbiological Laboratory Guidebook, no. 3, 9, 1 January 1998 (1998-01-01), pages 9-1-9-28, XP002596920, Retrieved from the Internet: URL:http://www.fsis.usda.gov/science/Micro biological_Lab_Guidebook/ [retrieved on 2010-08-17]
- ROSSMANITH ET AL: "Development of matrix lysis for concentration of gram positive bacteria from food and blood", JOURNAL OF MICROBIOLOGICAL METHODS, vol. 69, no. 3, 5 May 2007 (2007-05-05), pages 504-511, XP022059917, ELSEVIER, AMSTERDAM, NL ISSN: 0167-7012, DOI: 10.1016/J.MIMET.2007.03.003
- MAYRL EVA ET AL: "Broad Range Evaluation of the Matrix Solubilization (Matrix Lysis) Strategy for Direct Enumeration of Foodborne Pathogens by Nucleic Acids Technologies", JOURNAL OF FOOD PROTECTION, vol. 72, no. 6, 1 June 2009 (2009-06-01), pages 1225-1233, XP009137608, ISSN: 0362-028X
- FUKUSHIMA HIROSHI ET AL: "Rapid separation and concentration of food-borne pathogens in food samples prior to quantification by viable-cell counting and real-time PCR", APPLIED AND ENVIRONMENTAL MICROBIOLOGY, vol. 73, no. 1, January 2007 (2007-01), pages 92-100, XP002596921, ISSN: 0099-2240
- BENOIT P W ET AL: "METHODS FOR RAPID SEPARATION AND CONCENTRATION OF BACTERIA IN FOOD THAT BYPASS TIME-CONSUMING CULTURAL ENRICHMENT", JOURNAL OF FOOD PROTECTION, vol. 66, no. 10, 1 October 2003 (2003-10-01), pages 1935-1948, XP009056397, INTERNATIONAL ASSOCIATION FOR FOOD PROTECTION, US ISSN: 0362-028X
- STEVENS K A ET AL: "BACTERIAL SEPARATION AND CONCENTRATION FROM COMPLEX SAMPLE MATRICES: A REVIEW", CRITICAL REVIEWS IN MICROBIOLOGY, vol. 30, no. 1, 1 January 2004 (2004-01-01), pages 7-24, XP009056396, CRC PRESS ISSN: 1040-841X, DOI: 10.1080/10408410490266410
- MESTER PATRICK ET AL: "Use of Ionic Liquid-Based Extraction for Recovery of Salmonella Typhimurium and Listeria monocytogenes from Food Matrices", JOURNAL OF FOOD PROTECTION, vol. 73, no. 4, April 2010 (2010-04), pages 680-687, XP009137624, ISSN: 0362-028X
- SHELDON R: "Catalytic reactions in ionic liquids", CHEMICAL COMMUNICATIONS - CHEMCOM, no. 23, 18 October 2001 (2001-10-18), pages 2399-2407, XP002218386, ROYAL SOCIETY OF CHEMISTRY, GB ISSN: 1359-7345, DOI: 10.1039/B107270F
- VAN RANTWIJK F ET AL: "Biocatalysis in ionic liquids", CHEMICAL REVIEWS, vol. 107, no. 6, 13 June 2007 (2007-06-13) , pages 2757-2785, XP002590964, ACS,WASHINGTON, DC, US ISSN: 0009-2665, DOI: 10.1021/CR050946X [retrieved on 2007-06-13]

## Description

The present invention relates to a method and kit for the isolation of cells from a sample. The sample is treated with an extraction solution that comprises at least MgCl₂ resulting in the isolation of preferably viable cells.

### Background of the invention

The isolation of cells from complex samples for their identification or characterisation or simply for further processing is becoming increasingly important, in particular the identification of pathogens in samples like food samples or clinical samples like blood, tissue of feces. However, in order to clearly identify and optionally to quantify the cells comprised in a sample methods for their isolation have to be provided.

Real-time PCR has greatly enhanced the application field of PCR as a quantitative tool in molecular biology in general and for the quantification and identification of microorganisms, in particular of pathogens. Real-time PCR allows the reliable detection and quantification down to one single nucleic acid target per PCR reaction but requires highly purified template DNA. Especially when it comes to routine diagnostics and quantitative detection of bacteria in complex environments like food these requirements play a key role as inhibitory effects caused by components of these environments may influence or even inhibit the PCR reaction. Furthermore it is crucial to use a reliable and efficient recovery method to be used for the isolation of the target organisms from complex samples like food. Since samples like food involve generally large sample volumes microbiological methods are normally used for microorganism isolation and enrichment. These methods represent the "golden standard" methods and new alternative techniques have to be evaluated in comparison to them.

Major efforts have been made to establish methods for the separation of microorganisms, e.g. of bacteria, from food which meet the demanding requirements of real time PCR and other molecular methods for downstream analysis of the microorganisms.

Also the isolation of DNA directly out of food has been attempted using DNA isolation methods commonly used in molecular biology. Other methods utilize the affinity of biomolecules to surface structures of microorganisms, whereby said biomolecules may be, for instance, antibodies, bacteria binding proteins from phages and antimicrobial peptides (AMPs) optionally in combination with magnetic beads, silanized glass slides or direct colony blot. For instance, for the direct detection of Listeria monocytogenes an aqueous two-phase separation system can be used (Lantz et al. Appl Environ Microbiol. (1994) 60:3416-3418).

Buoyant density gradient centrifugation is reported as a tool for separation of bacteria from food matrices (Wolffs P . et al .Appl Environ Microbiol. (2005) 71:5759-5764). Other methods are based on physical separation such as simple centrifugation and filtration. Methods applying enzymatic digestion of the food matrix using proteinase K and pronase and/or chemical extraction of the bacteria from food using guanidine thiocyanate/phenol/chloroform, diethylether/chloroform, and sodium citrate/polyethylene glycol have also been described. Current methods for isolating cells, in particular microrganisms, from complex samples are described in, e.g., Stevens KA and Jaykus L-A (Crit Rev Microbiol (2004) 30:7-24).

Most of these methods have drawbacks like insufficient size of processed sample volume, high detection limits, low recovery rates, no quantitative isolation of viable cells, time consuming procedure and high costs. In addition the application of these methods has been restricted in most cases to only one or a limited number of different food matrices. Based on the requirements for direct quantification of bacteria in food which are (i) a large sample volume, (ii) a reproducible recovery rate over a broad range of target concentration, and (iii) removal of inhibitors to aid alternative molecular methods for downstream analysis, new protocols for separation of cells and microorganisms, like the food pathogen *L . monocytogenes*, have to be provided.

WO 2008/017097 discloses a method for isolating cells from complex matrices like foodstuff. This method uses an extraction buffer comprising a chaotropic agent in combination with a detergent.

Another key issue in food analysis is the determination of the viability of the contaminating bacteria. Up to now, most methods cannot distinguish between viable and dead bacterial cells. In addition to lyse the often complex matrix of food samples lysis conditions are needed which have negative impact on the viability of the cells to be isolated from such samples. This problem reduces the benefit of using e.g. PCR methods for routine monitoring in food analysis. On the one hand, some cells are killed during extraction, on the other hand metabolically injured or non-viable cells that have already been present in the sample before the extraction are also extracted and determined though they do not have any further effect on the quality of the sample.

O.F. D'Urso et al., Food Microbiology 26 (2009) 311-316 have developed a filtration-based method for isolating viable cells. The buffer used in this method comprises high amounts of the chaotrope guanidine thiocyanate which often interferes with downstream processes and thus has to be removed with complicated washing procedures.

Consequently, there exists a clear need for quantitative and reproducible methods for the isolation of cells from complex matrices like food and blood with the possibility to isolate viable cells.

### Brief description of the Invention

It has been found that cellular contaminants like bacteria can easily and very effectively be isolated from complex matrices using a buffer which comprises at least magnesium chloride (MgCl₂) and/or an ionic liquid. In addition, due to the very mild but effective extraction conditions, this method allows for the isolation of viable cells.

Therefore the present invention relates to a method for isolating bacterial cells from a complex sample comprising the steps of:
a) providing a complex sample,
b) incubating said sample with an extraction solution that comprises MgCl₂ in concentrations between 0.5 and 3 M
c) isolating said cells from the mixture of step b) by centrifugation, affinity binding and/or filtration.

### Description of the invention

It surprisingly turned out that the incubation of a complex sample with an extraction solution that comprises at least MgCl₂ and/or an ionic liquid results in the dissolution of the sample without affecting cells comprising or being surrounded with a cell wall contained in said sample. Therefore the method according to the present invention can suitably be employed for the isolation of such cells.

The term "cells surrounded by a cell wall" refers to all cells known having or comprising a cell wall as a barrier to the environment. Examples for organisms or cells having a cell wall are bacteria, archaea, fungi, plants and algae. In contrast thereto, animals and most other protists have cell membranes without surrounding cell walls.

The term "complex sample" refers to a sample or sample matrix comprising a greater or lesser number of different compounds of mainly organic origin, certain of which are liquid and others of which are solid. A complex sample according to the present invention may comprise a matrix comprising peptides, polypeptides, proteins (including also enzymes), carbohydrates (complex and simple carbohydrates), lipids, fatty acids, fat, nucleic acids etc.. The sample according to the present invention comprises preferably a low amount of fibers/starch.

As used herein, the term "sample with a low amount of fibers/starch" is used in a broad sense and is intended to include a variety of samples that contain or may contain cells.

Preferred samples comprise less than 20% (w/w), more preferably less than 10%, even more preferred less than 5%, especially preferred less than 1%, in particular no (under or around the detection limit), fibers/starch. "Fibers", as used herein, comprise fibers of plant as well as of animal (e.g. collagen fibres) origin.

Exemplary samples include, but are not limited to, food (e.g. milk of cows, ewes, nanny goats, mares, donkeys, camels, yak, water buffalo and reindeer, milk products, meat of beef, goat, lamb, mutton, pork, frog legs, veal, rodents, horse, kangaroo, poultry, including chicken, turkey, duck, goose, pigeon or dove, ostrich, emu, seafood, including finfish such as salmon and tilapia, and shellfish such as mollusks and crusta ceans and snails, meat products, plant products, seeds, cereals from grasses, including maize, wheat, rice, barley, sorghum, and millet, cereals from non-grasses, including buckwheat, amaranth, and quinoa, legumes, including beans, peanuts, peas, and lentils, nuts, including almonds, walnuts, and pine nuts, oilseeds, including sunflower, rape and sesame, vegetables like root vegetables, including potatoes, cassava and turnips, leaf vegetables, including amaranth, spinach and kale, sea vegetables, including dulse, kombu, and dabberlocks, stem vegetables, including bamboo shoots, nopales, and asparagus, inflorescence vegetables, including globe artichokes, broccoli, and daylilies, and fruit vegetables, including pumpkin, okra and eggplant, fruits, herbs and spices, whole blood, urine, sputum, saliva, amniotic fluid, plasma, serum, pulmonary lavage and tissues, including but not limited to, liver, spleen, kidney, lung, intestine, brain, heart, muscle, pancreas and the like. The skilled artisan will appreciate that lysates, extracts or (homogenized) material obtained from any of the above exemplary samples or mixtures of said exemplary samples or compositions comprising one or more of said exemplary samples are also samples within the scope of the invention.

The term "buffer" as used herein, refers to aqueous solutions or compositions that resist changes in pH when acids or bases are added to the solution or composition. This resistance to pH change is due to the buffering properties of such solutions. Thus, solutions or compositions exhibiting buffering activity are referred to as buffers or buffer solutions. Buffers generally do not have an unlimited ability to maintain the pH of a solution or composition. Rather, they are typically able to maintain the pH within certain ranges, for example between pH 7 and pH 9. Typically, buffers are able to maintain the pH within one log above and below their pKa (see, e.g. C . Mohan, Buffers, A guide for the preparation and use of buffers in biological systems, CALBIOCHEM, 1999). Buffers and buffer solutions are typically made from buffer salts or preferably from non-ionic buffer components like TRIS and HEPES. The buffer added to the extraction solution guarantees that the pH value in the course of the matrix dissolution will be stabilized. A stabilized pH value contributes to reproducible results, efficient lysis and conservation of the isolated cells.

According to a preferred embodiment of the present invention the isolated cells are viable cells.

It was surprisingly found that the cells isolated with the method according to the present invention are viable (at least 10%, preferably at least 30%, more preferably at least 50%, even more preferably at least 70%, most preferably at least 90% of the total intact cells isolated) and can be cultivated on suitable culture media.

As used herein, "viable cells" include cells with active metabolism, preferably propagable, especially cells which are able to multiply.

The cells to be isolated with the method according to the present invention are bacterial cells, preferably Gram-positive or Gram-negative bacterial cells, fungal cells, archaeal cells, algae cells or plant cells. Particularly preferred cells are selected from the group consisting of *Listeria spp., S. aureus, P. paratuberculosis*, *Salmonella spp., C. jejuni* and *Penicillum roquefortii.*

The method of the present invention allows the isolation of cells having or comprising a cell wall.

The present invention specifically allows isolation of microbial cells in general, preferably food and pathogen microbes, especially those of relevance for humans, e.g. those potentially present in human food or pathogens with clinical relevance. Therefore the method of the present invention allows to isolate bacterial cells, fungal cells, archaeal cells, algae cells and plant cells from a highly complex sample (e.g. food).

According to a preferred embodiment of the present invention the sample is a food sample, a body fluid, in particular blood, plasma or serum, water or a tissue sample.

Particularly preferred samples are samples with a complex matrix (i.e. comprising among others proteins, lipids, carbohydrates etc.) and/or a high viscosity.

The food sample is preferably a milk product, preferably milk, in particular raw milk, milk powder, yoghurt, cheese or ice cream, a fish product, preferably raw fish, a meat product, preferably raw meat, meat rinse or sausages, salad rinse, chocolate, egg or egg products, like mayonnaise.

Particularly preferred food samples used in the method according to the present invention are samples which are usually known to comprise potentially pathogenic organisms (e.g. *L*.*monocytogenes*) and from which cells are - due to a complex matrix - hardly extractable with the methods known in the art. In particular cheese is known as a food with a complex matrix and high viscosity.

According to the present invention, the extraction solution used as matrix lysis system comprises MgCl₂. The MgCl₂ is present in concentrations between 0.5 and 3 M, preferably between 0.5 and 2 M, more preferably between 1 and 2 M.

The ionic liquid - if present - is typically present in concentrations between 0.5 and 20% by weight, preferably between 1 and 10 % by weight, based on the weight of mixture. The ionic liquid can be one ionic liquid or a mixture of two or more ionic liquids.

The best concentration of the MgCl₂ and optionally the ionic liquid mainly depends on the sample to be dissolved and the cellular species to be isolated. These parameters can be tested easily by the person skilled in the art.

In a preferred embodiment, the extraction solution comprises either MgCl₂ or ionic liquid.

The extraction solution of the present invention is an aqueous solution or a buffer solution. It typically has a pH value greater than 5 and lower than 9, preferably greater than 6 and lower than 8, more preferably between 6.5 and 7.5. The extraction solution may additionally comprise up to 20% of one or more water-miscible organic solvents.

The buffer which may be used in the method of the present invention is preferably selected from the group of phosphate buffer, phosphate buffered saline buffer (PBS), 2-amino-2-hydroxymethyl-1,3-propanediol (TRIS) buffer, TRIS buffered saline buffer (TBS) and TRIS/EDTA (TE).

In contrast to known methods, according to the method of the present invention preferably no detergent, that means no anionic, zwitterionic or non-ionic detergent like sodium dodecylsulfate, CHAPS, Lutensol AO-7, is added to the extraction solution.

It is of course possible to add to the extraction solution one or more additional substances like destabilizing agents or biopolymer degrading enzymes which help to degrade substances present in specific samples. As discussed below, one example is the addition of starch degrading enzymes for food sample comprising high amounts of collagen and/or starch.

The incubation is typically performed at temperatures between 18°C and 50°C, preferably between 25°C and 45°C, more preferably between 30°C and 42°C.

The sample is typically incubated with the extraction solution for a time between 10 minutes and 6 hours, preferably between 20 minutes and 1 hours.

In order to dissolve the sample even more efficiently and in a reduced time, it is advantageous to perform the incubation at an elevated temperature. However, care should be taken that elevated temperatures may not affect - if desired - the viability of the cells to be isolated.

After incubation of the sample with the extraction solution and thus dissolution and lysis of the sample matrix the cells are isolated by centrifugation, filtration, or affinity binding, e.g. using antibodies, lectins, viral binding proteins, aptamers or antimicrobial peptides (AMP) which are preferably immobilized on beads. Preferably, the cells are isolated by filtration or centrifugation, most preferred by centrifugation.

Centrifugation is typically carried out at 500 to 10000 g, more preferably at 1500 to 6000 g, even more preferably at 2000 to 5000 g. After the centrifugation step the cells can be found in the pellet and the supernatant can be discarded.

If the sample/extraction solution mixture is filtered the cells are retained on the surface of said filter, when the pore size of the filter is adapted to the size of the cells to be isolated. Of course it is also possible to apply more than one filtration steps with different filters having varying pore sizes. After the filtration step the cells can be washed from the filter surface (see e.g. Stevens KA and Jaykus L-A, Crit Rev Microbiol (2004) 30:7-24). Filtration of the lysed sample is in particular required when the complex sample comprises material which will hardly or not be lysed with the method of the present invention.
Typically these materials comprise starch and/or fibers.

However, the preferred method for isolation the cells from the lysis mixture is centrifugation.

Of course it is also possible to isolate the cells from the dissolved pellet formed after the centrifugation step by immunological methods involving antibodies, in particular antibodies immobilized on beads, preferably magnetic beads, which are directed to epitopes present on the cells to be isolated. Since the use of antibody beads for isolating cells results in some cases in a reduced recovery rate, such methods may preferably employed mainly for qualitative isolation.

In order to facilitate the dissolution of the sample, said sample can be, for instance, homogenized using a stomacher prior its incubation with the extraction solution. The dissolution is further supported and/or accelerated when the sample/extraction solution mixture is agitated during the incubation.

The incubation step may - depending on the sample matrix - be repeated once or several times, e.g. twice, three times, four times, five times or ten times. Between these incubation steps the cells and the remnant sample matrix may be separated from the supernatant by e.g. centrifugation.

The cells isolated with the method according to the present invention may be used for quantitatively or qualitatively determining the cells in the sample. This can be achieved, for instance, by cell counting, by PCR methods, in particular by real time PCR, by using lectins or by methods involving antibodies, viral binding proteins, aptamers or antimicrobial peptides (AMP) directed to surface structures of said cells (e.g. cell specific ELISA or RIA).

After the isolation step the cells are preferably washed with water, a buffer solution and/or detergent comprising solutions. However, it is of course possible to add to the wash buffer one or more additional substances. The wash step may be repeated for several times (e.g. 2, 3, 4 , 5 or 10 times) or only once. In the course of the washing step the cells are typically resuspended in the buffer and then filtered or centrifuged. If insoluble particles are present in the dissolved sample (e.g. calcium phosphate particles of cheese) said particles can be removed either by centrifugation at a lower rotational speed or by letting the particles settle over time (cells will remain in both cases in the supernatant) .

The cells may also be washed with detergent comprising solutions. This will allow to further remove fat remnants potentially contained in the cell suspension. Preferred detergents to be used in this method step are those detergents regularly used for fat removal.

One advantage of the method according to the present invention is that the extraction solution only comprises MgCl₂ in moderate concentrations but no detergents. As a consequence, in contrast to known methods where the extraction buffer typically comprises detergents and high amounts of chaotropes, it is possible to leave out or significantly reduce the washing steps if the sample matrix allows for it, that means if it does not comprise e.g. fat remnants that need to be removed with detergent comprising wash buffers. This feature of the present method makes it possible to reduce extraction time and to directly or at least nearly directly after only one or two washing steps analyze the cells with methods (like ELISA) which would otherwise be disturbed by the presence of chaotropic substances or detergents.

Due to the fact that preferably no detergent is present in the extraction solution, it is also possible to directly isolate the cells using antibodies bound preferably to a solid support (e.g. beads, in particular magnetic beads). The binding of the cells to antibodies permits to specifically isolate a certain type of cells. This is especially of interest when the sample comprises more than one cell species.

According to a preferred embodiment of the present invention the amount of the cells in the sample is determined.

The amount of the cells in the sample can be determined by any method known in the art, in particular by microbiological methods (e.g. dilution series), cell count, FACS analysis, real time PCR etc..

According to another preferred embodiment of the present invention the DNA or RNA of the cells is isolated.

Depending on the cells various methods may be employed to extract DNA (e.g. genomic DNA, plasmids) or RNA (e.g. mRNA). All these methods are known in the art and the single protocols mainly depend on the cell to be lysed. The isolation may further require the addition of enzymes like lysozyme.

In order to enhance the lysis of the samples, in particular of samples with a high viscosity (e.g. cheese), said sample is processed by a stomacher or mixer prior incubation with the extraction solution.

In order to determine or to monitor the efficiency of the isolation procedure the sample can be spiked with a defined amount of control cells. The control cells are typically bacterial cells, preferably Gram-positive or Gram-negative bacterial cells, fungal cells, archaeal cells, algae cells or plant cells. Preferably they are similar to the cells assumed to be present in the sample but they are preferably not identical to the cells assumed to be present in the sample. The amount of the recovered spiked control cells allows to determine the efficiency of the method of the present invention and may also indicate the amount of the cells to be isolated and determined present in the initial sample.

It is also possible to preincubate the sample with a compound exhibiting osmotic stress protective properties to the cells.

In order to increase the resistance of the cells against osmotic stress, the sample comprising (potentially) the cells to be isolated may be incubated with at least one compound which is able to induce osmotic protective responses in said cells.

Compounds exhibiting such characteristics and which are preferably used in the method of the present invention are glycine betaine and/or betalysine.

According to one embodiment of the present invention the sample is further incubated with at least one biopolymer degrading enzyme.

Some samples from which the cells are isolated comprise structures of biopolymers which may not or only in an inefficient manner be lysed by the addition of the extraction solution. If the sample, in particular the food sample, for example comprises collagen and/or starch in an amount of, e.g., over 10%, said sample may be treated with substances capable of degrading at least partially the collagen and starch content prior to its incubation with the matrix lysis system of the present invention.

Therefore the sample is preferably incubated further with at least one biopolymer degrading enzyme. Samples which are preferably incubated with biopolymer degrading enzymes are e.g. meat, fish, etc. Ice cream, eggs, blood, milk, milk products etc. do usually not require the addition of biopolymer degrading enzyme. It surprisingly turned out that the use of enzymes alone does not allow the isolation of cells.

As used herein, the term "biopolymer" refers to proteins, polypeptides, nucleic acids, polysaccharides like cellulose, starch and glycogen etc. Therefore a "biopolymer degrading enzyme" is an enzyme which is able to degrade a biopolymer (e.g. starch, cellulose), which may be insoluble in an aqueous buffer, to low molecular substances or even to monomers. Since the biopolymer degrading enzyme may be active under certain pH and temperature conditions (the use of specific buffers may also play a role) it is advantageous to perform the incubation with said enzymes under optional conditions. These conditions depend on the enzyme used and are known in the art. Also the incubation time depends on extrinsic factors like pH and temperature. Therefore the incubation time may vary from 10s to 6h, preferably 30s to 2h.

The biopolymer degrading enzyme is preferably selected from the group consisting of proteases, cellulases and amylase. Examples of these enzymes are Savinase 24 GTT (Subtilin), Carenzyme 900 T , Stainzyme GT. Starch degrading enzymes are e.g. cyclodextrin glucanotransferase, alpha-amylase, beta-amylase, glucoamylase, pullulanase and isoamylase, in particular α-amylase.

In known methods using buffers comprising chaotropic agents and detergents the biopolymer degrading enzymes cannot be added during the matrix lysis step as chaotropes and detergents may negatively influence the enzyme activity so that the biopolymers are not efficiently degraded into fragments or monomers.

In contrast to this, in the method according to the present invention, the biopolymer degrading enzyme can be incubated with the sample prior to step b) and/or during step b) and/or after step c) (step b) being the lysis step where the sample is incubated with the extraction solution and step c) being the isolation step).

The method according to the present invention can be performed within a few hours, typically within 1 to 6 hours.

Ionic liquids or liquid salts are ionic species which consist of an organic cation and a generally inorganic anion. They do not contain any neutral molecules and usually have melting points below 373 K.

The area of ionic liquids is currently being researched intensively since the potential applications are multifarious. Review articles on ionic liquids are, for example, R. Sheldon "Catalytic reactions in ionic liquids", Chem. Commun., 2001. 2399-2407; M.J. Earle, K.R. Seddon "Ionic liquids. Green solvent for the future", Pure Appl. Chem., 72 (2000), 1391-1398; P.

Wasserscheid, W. Keim "Ionische Flussigkeiten - neue Lösungen für die Übergangsmetallkatalyse" [Ionic Liquids - Novel Solutions for Transition-Metal Catalysis], Angew. Chem., 112 (2000), 3926-3945; T. Welton "Room temperature ionic liquids. Solvents for synthesis and catalysis", Chem. Rev., 92 (1999), 2071-2083 or R. Hagiwara, Ya. Ito "Room temperature ionic liquids of alkylimidazolium cations and fluoroanions", J. Fluorine Chem., 105 (2000), 221-227).

In general, all ionic liquids of the general formula K⁺A⁻ known to the person skilled in the art, in particular those which are miscible with water, are suitable in the method according to the invention.

The anion A⁻ of the ionic liquid is preferably selected from the group comprising halides, tetrafluoroborate, hexafluorophosphate, cyanamide, thiocyanate or imides of the general formula [N(R_{f})₂]⁻ or of the general formula [N(XR_{f})₂]⁻, where R_{f} denotes partially or fully fluorine-substituted alkyl having 1 to 8 C atoms and X denotes SO₂ or CO. The halide anions here can be selected from chloride, bromide and iodide anions, preferably from chloride and bromide anions. The anions A⁻ of the ionic liquid are preferably halide anions, in particular bromide or iodide anions, or tetrafluoroborate or cyanamide or thiocyanate.

There are no restrictions per se with respect to the choice of the cation K⁺ of the ionic liquid. However, preference is given to organic cations, particularly preferably ammonium, phosphonium, uronium, thiouronium, guanidinium cations or heterocyclic cations.

Ammonium cations can be described, for example, by the formula (1)

[NR₄]⁺ (1),

where
R in each case, independently of one another, denotes
H, where all substituents R cannot simultaneously be H, OR', NR'₂, with the proviso that a maximum of one substituent R in formula (1) is OR', NR'₂,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more R may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' may be = H, non-, partially or perfluorinated C₁- to C₆-alkyl, C₃- to C₇-cycloalkyl, unsubstituted or substituted phenyl and X may be = halogen.

Phosphonium cations can be described, for example, by the formula (2)

[PR²₄]⁺ (2),

where
R² in each case, independently of one another, denotes
H, OR' or NR'₂
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more R² may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R² which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

However, cations of the formulae (1) and (2) in which all four or three substituents R and R² are fully substituted by halogens are excluded, for example the tris(trifluoromethyl)methylammonium cation, the tetra(trifluoromethyl)ammonium cation or the tetra(nonafluorobutyl)ammonium cation.

Uronium cations can be described, for example, by the formula (3)

[(R³R⁴N)-C(=OR⁵)(NR⁶R⁷)]⁺ (3),

and thiouronium cations by the formula (4),

[(R³R⁴N)-C(=SR⁵)(NR⁶R⁷)]⁺ (4),

where
R³ to R⁷ each, independently of one another, denotes
hydrogen, where hydrogen is excluded for R⁵,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R³ to R⁷ may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R³ to R⁷ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

Guanidinium cations can be described by the formula (5)

[C(NR⁸R⁹)(NR¹⁰R¹¹)(NR¹²R¹³)]⁺ (5),

where
R⁸ to R¹³ each, independently of one another, denotes
hydrogen, -CN, NR'₂, -OR'
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms, where one or more of the substituents R⁸ to R¹³ may be partially or fully substituted by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂, and where one or two non-adjacent carbon atoms in R⁸ to R¹³ which are not in the α-position may be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

In addition, it is possible to employ cations of the general formula (6)

[HetN]⁺ (6),

where
HetN⁺ denotes a heterocyclic cation selected from the group 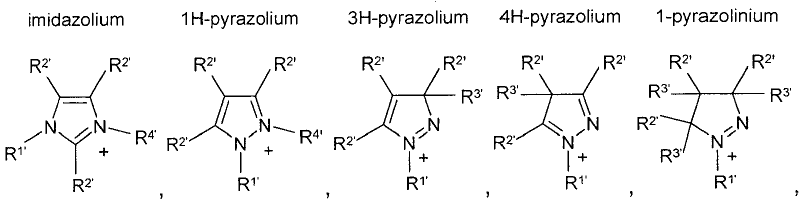 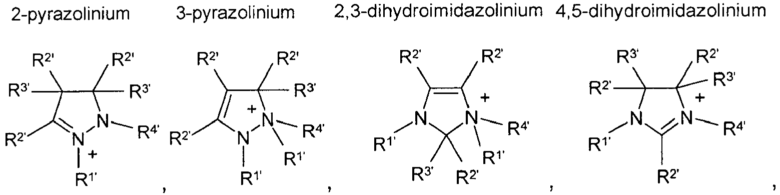 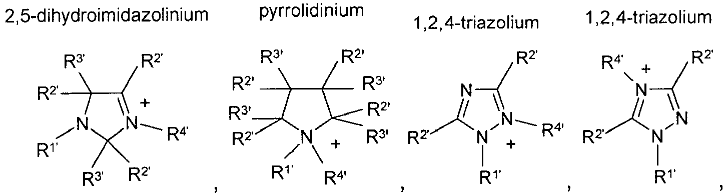 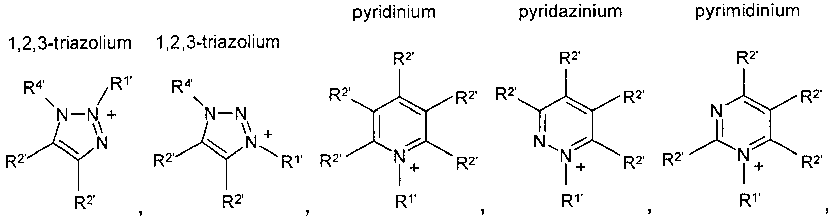 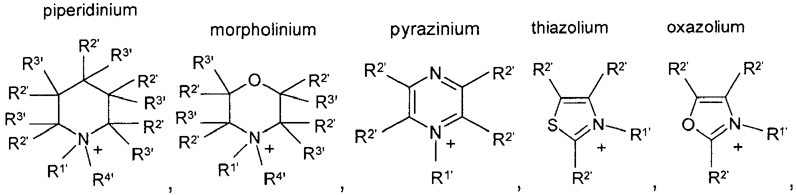 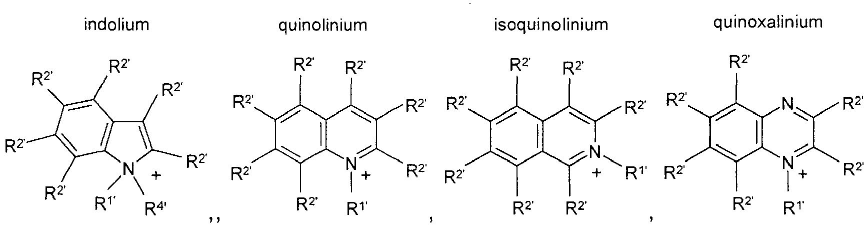 or 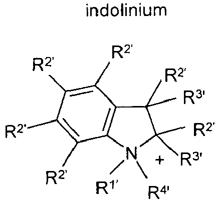 where the substituents
R¹' to R⁴' each, independently of one another, denote
hydrogen, -CN, -OR', -NR'₂, -P(O)R'₂, -P(O)(OR')₂, -P(O)(NR'₂)₂, -C(O)R', -C(O)OR',
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
saturated, partially or fully unsaturated heteroaryl, heteroaryl-C₁-C₆-alkyl or aryl-C₁-C₆-alkyl,
where the substituents R^{1'}, R^{2'}, R^{3'} and/or R^{4'} together may also form a ring system,
where one or more substituents R^{1'} to R^{4'} may be partially or fully substituted by halogens, in particular -F and/or -Cl, or -OH, -OR', -CN, -C(O)OH, -C(O)NR'₂, -SO₂NR'₂, -C(O)X, -SO₂OH, -SO₂X, -NO₂, but where R^{1'} and R^{4'} cannot simultaneously be fully substituted by halogens, and where, in the substituents R¹' to R⁴', one or two non-adjacent carbon atoms which are not bonded to the heteroatom may be replaced by atoms and/or atom groups selected from the -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = H, non-, partially or perfluorinated C₁- to C₆-alkyl, C₃- to C₇-cycloalkyl, unsubstituted or substituted phenyl and X = halogen.

For the purposes of the present invention, fully unsaturated substituents are also taken to mean aromatic substituents.

In accordance with the invention, suitable substituents R and R² to R¹³ of the compounds of the formulae (1) to (5), besides hydrogen, are preferably: C₁- to C₂₀-, in particular C₁- to C₁₄-alkyl groups, and saturated or unsaturated, i.e. also aromatic, C₃- to C₇-cycloalkyl groups, which may be substituted by C₁- to C₆-alkyl groups, in particular phenyl.

The substituents R and R² in the compounds of the formula (1) or (2) may be identical or different here. The substituents Rand R² are preferably different.

The substituents R and R² are particularly preferably methyl, ethyl, isopropyl, propyl, butyl, sec-butyl, tert-butyl, pentyl, hexyl, octyl, decyl or tetradecyl.

Up to four substituents of the guanidinium cation [C(NR⁸R⁹)(NR¹⁰R¹¹)(NR¹²R¹³)]⁺ may also be bonded in pairs in such a way that mono-, bi- or polycyclic cations are formed.
Without restricting generality, examples of such guanidinium cations are: 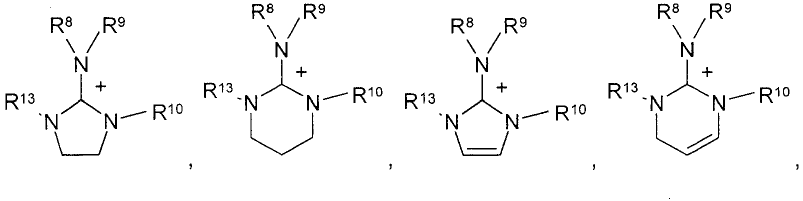 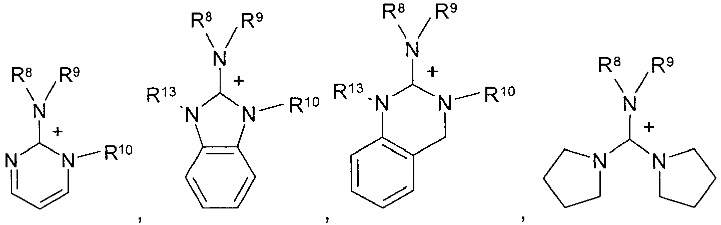 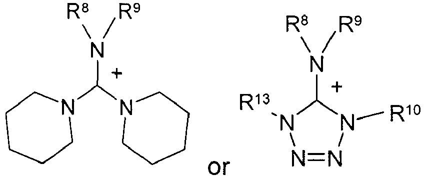 where the substituents R⁸ to R¹⁰ and R¹³ can have a meaning or particularly preferred meaning indicated above.
If desired, the carbocyclic or heterocyclic rings of the guanidinium cations indicated above may also be substituted by C₁- to C₆-alkyl, C₁- to C₆-alkenyl, NO₂, F, Cl, Br, I, OH, C₁-C₆-alkoxy, SCF₃, SO₂CF₃, COOH, SO₂NR'₂, SO₂X' or SO₃H, where X and R' have a meaning indicated above, substituted or unsubstituted phenyl or an unsubstituted or substituted heterocycle.

Up to four substituents of the uronium cation [(R³R⁴N)-C(=OR⁵)(NR⁶R⁷)]⁺ or thiouronium cation [(R³R⁴N)-C(=SR⁵)(NR⁶R⁷)]⁺ may also be bonded in pairs in such a way that mono-, bi- or polycyclic cations are formed.

Without restricting generality, examples of such cations are indicated below, where Y = O or S: 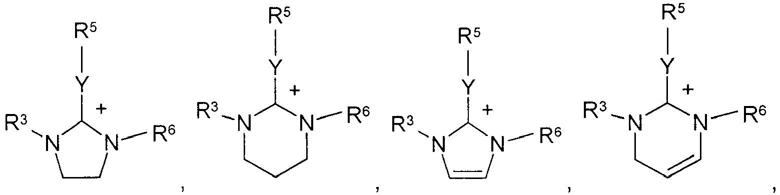 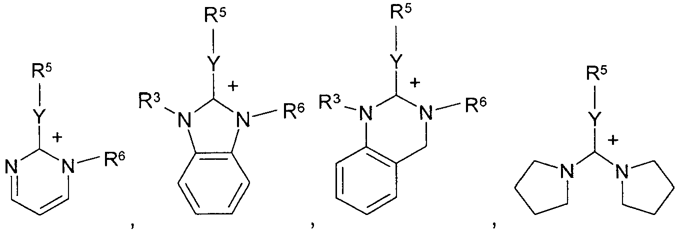 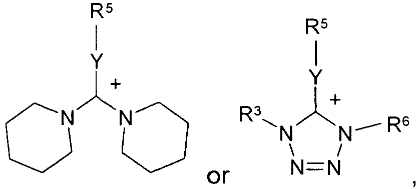 where the substituents R³, R⁵ and R⁶ can have a meaning or particularly preferred meaning indicated above.
If desired, the carbocyclic or heterocyclic rings of the cations indicated above may also be substituted by C₁- to C₆-alkyl, C₁- to C₆-alkenyl, NO₂, F, Cl, Br, I, OH, C₁-C₆-alkoxy, SCF₃, SO₂CF₃, COOH, SO₂NR'₂, SO₂X or SO₃H or substituted or unsubstituted phenyl or an unsubstituted or substituted heterocycle, where X and R' have a meaning indicated above.

The substituents R³ to R¹³ are each, independently of one another, preferably a straight-chain or branched alkyl group having 1 to 10 C atoms. The substituents R³ and R⁴, R⁶ and R⁷, R⁸ and R⁹, R¹⁰ and R¹¹ and R¹² and R¹³ in compounds of the formulae (3) to (5) may be identical or different. R³ to R¹³ are particularly preferably each, independently of one another, methyl, ethyl, n-propyl, isopropyl, n-butyl, tert-butyl, sec-butyl, phenyl or cyclohexyl, very particularly preferably methyl, ethyl, n-propyl, isopropyl or n-butyl.

In accordance with the invention, suitable substituents R^{1'} to R^{4'} of compounds of the formula (6), besides hydrogen, are preferably: C₁- to C₂₀, in particular C₁- to C₁₂-alkyl groups, and saturated or unsaturated, i.e. also aromatic, C₃- to C₇-cycloalkyl groups, which may be substituted by C₁- to C₆-alkyl groups, in particular phenyl.

The substituents R^{1'} and R^{4'} are each, independently of one another, particularly preferably methyl, ethyl, isopropyl, propyl, butyl, sec-butyl, tert-butyl, pentyl, hexyl, octyl, decyl, cyclohexyl, phenyl or benzyl. They are very particularly preferably methyl, ethyl, n-butyl or hexyl. In pyrrolidinium, piperidinium or indolinium compounds, the two substituents R^{1'} and R^{4'} are preferably different.

The substituent R^{2'} or R^{3'} is in each case, independently of one another, in particular hydrogen, methyl, ethyl, isopropyl, propyl, butyl, sec-butyl, tert-butyl, cyclohexyl, phenyl or benzyl. R^{2'} is particularly preferably hydrogen, methyl, ethyl, isopropyl, propyl, butyl or sec-butyl. R^{2'} and R^{3'} are very particularly preferably hydrogen.
The C₁-C₁₂-alkyl group is, for example, methyl, ethyl, isopropyl, propyl, butyl, sec-butyl or tert-butyl, furthermore also pentyl, 1-, 2- or 3-methylbutyl, 1,1-, 1,2- or 2,2-dimethylpropyl, 1-ethylpropyl, hexyl, heptyl, octyl, nonyl, decyl, undecyl or dodecyl. Optionally difluoromethyl, trifluoromethyl, pentafluoroethyl, heptafluoropropyl or nonafluorobutyl.

A straight-chain or branched alkenyl having 2 to 20 C atoms, in which a plurality of double bonds may also be present, is, for example, allyl, 2- or 3-butenyl, isobutenyl, sec-butenyl, furthermore 4-pentenyl, isopentenyl, hexenyl, heptenyl, octenyl, -C₉H₁₇, -C₁₀H₁₉ to -C₂₀H₃₉; preferably allyl, 2- or 3-butenyl, isobutenyl, sec-butenyl, furthermore preferably 4-pentenyl, isopentenyl or hexenyl.

A straight-chain or branched alkynyl having 2 to 20 C atoms, in which a plurality of triple bonds may also be present, is, for example, ethynyl, 1- or 2-propynyl, 2- or 3-butynyl, furthermore 4-pentynyl, 3-pentynyl, hexynyl, heptynyl, octynyl, -C₉H₁₅, -C₁₀H₁₇ to -C₂₀H₃₇, preferably ethynyl, 1- or 2-propynyl, 2- or 3-butynyl, 4-pentynyl, 3-pentynyl or hexynyl.

Aryl-C₁-C₆-alkyl denotes, for example, benzyl, phenylethyl, phenylpropyl, phenylbutyl, phenylpentyl or phenylhexyl, where both the phenyl ring and also the alkylene chain may be partially or fully substituted, as described above, by halogens, in particular -F and/or -Cl, or partially by -OH, -OR', -CN, -C(O)OH, -C(O)NR'₂, -SO₂NR'₂, -C(O)X, -SO₂OH, -SO₂X, -NO₂.

Unsubstituted saturated or partially or fully unsaturated cycloalkyl groups having 3-7 C atoms are therefore cyclopropyl, cyclobutyl, cyclopentyl, cyclohexyl, cycloheptyl, cyclopentenyl, cyclopenta-1,3-dienyl, cyclohexenyl, cyclohexa-1,3-dienyl, cyclohexa-1,4-dienyl, phenyl, cycloheptenyl, cyclo-hepta-1,3-dienyl, cyclohepta-1,4-dienyl or cyclohepta-1,5-dienyl, each of which may be substituted by C₁- to C₆-alkyl groups, where the cycloalkyl group or the cycloalkyl group substituted by C₁- to C₆-alkyl groups may in turn also be substituted by halogen atoms, such as F, Cl, Br or I, in particular F or Cl, or by -OH, -OR', -CN, -C(O)OH, -C(O)NR'₂, -SO₂NR'₂, -C(O)X, -SO₂OH, -SO₂X, -NO₂.

In the substituents R, R² to R¹³ or R^{1'} to R^{4'}, one or two non-adjacent carbon atoms which are not bonded in the α-position to the heteroatom may also be replaced by atoms and/or atom groups selected from the group -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- or -P(O)R'- where R' = non-, partially or perfluorinated C₁- to C₆-alkyl, C₃- to C₇-cycloalkyl, unsubstituted or substituted phenyl.

Without restricting generality, examples of substituents R, R² to R¹³ and R^{1'} to R^{4'} modified in this way are:
-OCH₃, -OCH(CH₃)₂, -CH₂OCH₃, -CH₂-CH₂-O-CH₃, -C₂H₄OCH(CH₃)₂, -C₂H₄SC₂H₅, -C₂H₄SCH(CH₃)₂, -S(O)CH₃, -SO₂CH₃, -SO₂C₆H₅, -SO₂C₃H₇, -SO₂CH(CH₃)₂, -SO₂CH₂CF₃, -CH₂SO₂CH₃, -O-C₄H₈-O-C₄H₉, -CF₃, -C₂F₅, -C₃F₇, -C₄F₉, -C(CF₃)₃, -CF₂SO₂CF₃, -C₂F₄N(C₂F₅)C₂F₅, -CHF₂, -CH₂CF₃, -C₂F₂H₃, -C₃FH₆, -CH₂C₃F₇, -C(CFH₂)₃, -CH₂C(O)OH, -CH₂C₆H₅, -C(O)C₆H₅ or P(O)(C₂H₅)₂.

In R', C₃- to C₇-cycloalkyl is, for example, cyclopropyl, cyclobutyl, cyclopentyl, cyclohexyl or cycloheptyl.

In R', substituted phenyl denotes phenyl which is substituted by C₁- to C₆-alkyl, C₁- to C₆-alkenyl, NO₂, F, Cl, Br, I, OH, C₁-C₆-alkoxy, SCF₃, SO₂CF₃, COOH, SO₂X', SO₂NR"₂ or SO₃H, where X' denotes F, Cl or Br and R" denotes a non-, partially or perfluorinated C₁- to C₆-alkyl or C₃- to C₇-cycloalkyl as defined for R', for example o-, m- or p-methylphenyl, o-, m- or p-ethylphenyl, o-, m- or p-propylphenyl, o-, m- or p-isopropylphenyl, o-, m- or p-tert-butylphenyl, o-, m- or p-nitrophenyl, o-, m- or p-hydroxyphenyl, o-, m- or p-methoxyphenyl, o-, m- or p-ethoxyphenyl, o-, m-, p-(trifluoromethyl)-phenyl, o-, m-, p-(trifluoromethoxy)phenyl, o-, m-, p-(trifluoromethylsulfonyl)phenyl, o-, m- or p-fluorophenyl, o-, m- or p-chlorophenyl, o-, m- or p-bromophenyl, o-, m- or p-iodophenyl, further preferably 2,3-, 2,4-, 2,5-, 2,6-, 3,4- or 3,5-dimethylphenyl, 2,3-, 2,4-, 2,5-, 2,6-, 3,4- or 3,5-dihydroxyphenyl, 2,3-, 2,4-, 2,5-, 2,6-, 3,4- or 3,5-difluorophenyl, 2,3-, 2,4-, 2,5-, 2,6-, 3,4- or 3,5-dichlorophenyl, 2,3-, 2,4-, 2,5-, 2,6-, 3,4- or 3,5-dibromophenyl, 2,3-, 2,4-, 2,5-, 2,6-, 3,4- or 3,5-dimethoxyphenyl, 5-fluoro-2-methylphenyl, 3,4,5-trimethoxyphenyl or 2,4,5-trimethylphenyl.

In R^{1'} to R^{4'}, heteroaryl is taken to mean a saturated or unsaturated mono- or bicyclic heterocyclic radical having 5 to 13 ring members, in which 1, 2 or 3 N and/or 1 or 2 S or O atoms may be present and the heterocyclic radical may be mono- or polysubstituted by C₁- to C₆-alkyl, C₁- to C₆-alkenyl, NO₂, F, Cl, Br, I, OH, C₁-C₆-alkoxy, SCF₃, SO₂CF₃, COOH, SO₂X', SO₂NR"₂ or SO₃H, where X' and R" have a meaning indicated above.

The heterocyclic radical is preferably substituted or unsubstituted 2- or 3-furyl, 2- or 3-thienyl, 1-, 2- or 3-pyrrolyl, 1-, 2-, 4- or 5-imidazolyl, 3-, 4- or 5-pyrazolyl, 2-, 4- or 5-oxazolyl, 3-, 4- or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, furthermore preferably 1,2,3-triazol-1-, -4- or -5-yl, 1,2,4-triazol-1-, -4- or -5-yl, 1- or 5-tetrazolyl, 1,2,3-oxadiazol-4- or -5-yl 1,2,4-oxadiazol-3- or -5-yl, 1,3,4-thiadiazol-2- or -5-yl, 1,2,4-thiadiazol-3- or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-1H-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5-, 6- or 7-benzisoxazolyl, 2-, 4-, 5-, 6- or 7-benzothiazolyl, 2-, 4-, 5-, 6- or 7-benzisothiazolyl, 4-, 5-, 6- or 7-benz-2,1,3-oxadiazolyl, 1-, 2-, 3-, 4-, 5-, 6-, 7- or 8-quinolinyl, 1-, 3-, 4-, 5-, 6-, 7- or 8-isoquinolinyl, 1-, 2-, 3-, 4- or 9-carbazolyl, 1-, 2-, 3-, 4-, 5-, 6-, 7-, 8- or 9-acridinyl, 3-, 4-, 5-, 6-, 7- or 8-cinnolinyl, 2-, 4-, 5-, 6-, 7- or 8-quinazolinyl or 1-, 2- or 3-pyrrolidinyl.

Heteroaryl-C₁-C₆-alkyl is, analogously to aryl-C₁-C₆-alkyl, taken to mean, for example, pyridinylmethyl, pyridinylethyl, pyridinylpropyl, pyridinylbutyl, pyridinylpentyl, pyridinylhexyl, where the heterocyclic radicals described above may furthermore be linked to the alkylene chain in this way.

HetN⁺ is preferably 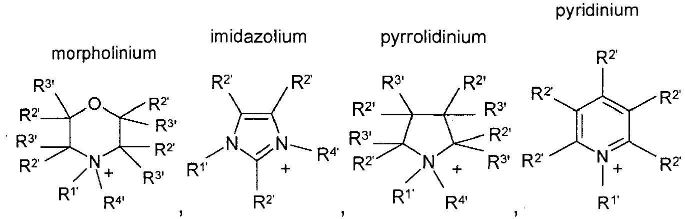 or 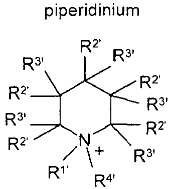 where the substituents R^{1'} to R^{4'} each, independently of one another, have a meaning described above. Morpholinium and imidazolium cations are particularly preferred in the present invention, where R¹' to R⁴' in the said cations denote, in particular, in each case independently of one another, hydrogen, straight-chain or branched alkyl having 1-20 C atoms, where one or more substituents R¹' to R⁴' may be partially substituted by -OH or -OR', where R¹' = non-, partially or perfluorinated C₁- to C₆-alkyl, C₃- to C₇-cycloalkyl, unsubstituted or substituted phenyl.

The cations of the ionic liquid according to the invention are preferably ammonium, phosphonium, imidazolium or morpholinium cations, most preferred are imidazolium cations.

Very particularly preferred substituents R, R², R¹' to R⁴' of the preferred ammonium, phosphonium, imidazolium or morpholinium cations are selected from methyl, ethyl, propyl, butyl, hexyl, decyl, dodecyl, octadecyl, ethoxyethyl, methoxyethyl, hydroxyethyl or hydroxypropyl groups.

It is preferred that the imidazolium cations are substituted by alkyl, alkenyl, aryl and/or aralkyl groups which may themselves be substituted by functional groups such as by groups containing nitrogen, sulfur and/or phosphorous wherein different oxidation states are possible. Preferred examples of these functional groups according to the invention are: amine, carboxyl, carbonyl, aldehyde, hydroxy, sulfate, sulfonate and/or phosphate groups.

One or both of the N atoms of the imidazolium ring can be substituted by identical or different substituents. Preferably both nitrogen atoms of the imidazolium ring are substituted by identical or different substituents.

It is also possible or preferred according to the invention that the imidazolium salts are additionally or exclusively substituted at one or more of the carbon atoms of the imidazolium ring.

Preferred as the substituents are C₁-C₄ alkyl groups such as methyl, ethyl, n-propyl, isopropyl, n-butyl and/or isobutyl groups. Substituents which are also preferred are C₂-C₄ alkenyl groups such as ethylene, n-propylene, isopropylene, n-butylene and/or isobutylene, also alkyl and alkenyl substituents having more than 4 C atoms are comprised wherein for example also C₅-C₁₀ alkyl or alkenyl substituents are still preferred. Due to solubility of the ionic liquid it might be favourable that these C₅-C₁₀ alkyl or alkenyl groups have one or more other substituents such as phosphate, sulfonate, amino and/or phosphate groups at their alkyl and/or alkenyl groups.

As the aryl substituents are preferred according to the invention mono-and/or bicyclic aryl groups, phenyl, biphenyl and/or naphthalene as well as derivatives of these compounds which carry hydroxy, sulfonate, sulfate, amino, aldehyde, carbonyl and/or carboxy groups. Examples of preferred aryl substituents are phenol, biphenyl, biphenol, naphthalene, naphthalene carboxylic acids, naphthalene sulfonic acids, biphenylols, biphenyl carboxylic acids, phenol, phenyl sulfonate and/or phenol sulfonic acids.

Imidazolium thiocyanates, dicyanamides, tetrafluoroborates, iodides, chlorides, bromides or hexafluorophosphates are very particularly preferably employed in the methods according to the invention, where 1-decyl-3-methylimidazolium bromide, 1-decyl-3-methylimidazolium iodide, 1-decyl-3-methylimidazolium hexafluorophosphate, 1-decyl-3-methylimidazolium tetrafluoroborate, 1-decyl-3-methylimidazolium thiocyanate, 1-decyl-3-methylimidazolium dicyanamide, 1-dodecyl-3-methylimidazolium chloride, 1-dodecyl-3-methylimidazolium bromide, 1-dodecyl-3-methylimidazolium iodide, 1-dodecyl-3-methylimidazolium hexafluorophosphate, 1-dodecyl-3-methylimidazolium tetrafluoroborate, 1-dodecyl-3-methylimidazolium thiocyanate, 1-dodecyl-3-methylimidazolium dicyanamide, 1-hexyl-3-methylimidazolium bromide, 1-hexyl-3-methylimidazolium chloride, 1-hexyl-3-methylimidazolium iodide, 1-hexyl-3-methylimidazolium hexafluorophosphate, 1-hexyl-3-methylimidazolium tetrafluoroborate, 1-hexyl-3-methylimidazolium thiocyanate, 1-hexyl-3-methylimidazolium dicyanamide, 1-octyl-3-methylimidazolium bromide, 1-octyl-3-methylimidazolium iodide, 1-octyl-3-methylimidazolium hexafluorophosphate, 1-octyl-3-methylimidazolium tetrafluoroborate, 1-octyl-3-methylimidazolium thiocyanate, 1-octyl-3-methylimidazolium dicyanamide, 1-butyl-3-methylimidazolium bromide, 1-butyl-3-methylimidazolium iodide, 1-butyl-3-methylimidazolium hexafluorophosphate, 1-butyl-3-methylimidazolium tetrafluoroborate, 1-butyl-3-methylimidazolium thiocyanate, 1-butyl-3-methylimidazolium dicyanamide, 1-ethyl-3-methylimidazolium bromide, 1-ethyl-3-methylimidazolium iodide, 1-ethyl-3-methylimidazolium hexafluorophosphate, 1-ethyl-3-methylimidazolium tetrafluoroborate, 1-ethyl-3-methylimidazolium thiocyanate, 1-ethyl-3-methylimidazolium dicyanamide, are especially preferred in the method according to the invention. Most preferred are 1-butyl-3-methylimidazolium tetrafluoroborate, 1-butyl-3-methylimidazolium thiocyanate, 1-butyl-3-methylimidazolium dicyanamide, 1-ethyl-3-methylimidazolium tetrafluoroborate, 1-ethyl-3-methylimidazolium thiocyanate, 1-ethyl-3-methylimidazolium dicyanamide, 1-hexyl-3-methylimidazolium tetrafluoroborate, 1-hexyl-3-methylimidazolium thiocyanate, 1-hexyl-3-methylimidazolium dicyanamide.

The ionic liquids are preferably liquids, i.e. preferably they are liquids which are ionic at room temperature (about 25° C.). However, also ionic liquids can be used which are not liquid at room temperature but which then should be present in a liquid form or should be soluble in the extraction solution at the temperature at which the method of the present invention is performed.

Also disclosed is an extraction solution for the isolation of cells from a complex matrix comprising at least:
- MgCl₂ and/or an ionic liquid
   typically in water or an aqueous buffer.

The MgCl₂ - if present - is typically present in the extraction solution in concentrations between 0.5 and 3 M, preferably between 0.5 and 2 M, more preferably between 1 and 2 M.

The ionic liquid - if present - is typically present in concentrations between 0.5 and 20% by weight, preferably between 1 and 10 % by weight, based on the mixture.

According to a preferred embodiment the extraction solution has a pH value greater than 5 and lower than 9, preferably greater than 6 and lower than 8, more preferably between 6.5 and 7.5.

The buffer of the present invention is selected from the group of phosphate buffer, phosphate buffered saline buffer (PBS), 2-amino-2-hydroxymethyl-1, 3-propanediol (TRIS) buffer, TRIS buffered saline buffer (TBS) and TRIS/EDTA (TE).

Also disclosed is a kit for the isolation of cells from a complex matrix comprising:
- an extraction solution according to the present invention and
- at least one biopolymer degrading enzyme (see above).

According to a preferred embodiment the at least one biopolymer degrading enzyme is selected from the group consisting of proteases, cellulases and amylases, preferably α-amylases.

The method according to the present invention offers a very mild and effective matrix lysis system. The extraction solution effectively lyses the matrix of most of the complex samples which are e.g. typical in food analysis while the target cells remain unaffected and thus viable. Due to the very mild matrix lysis conditions, even the surface structures of the cells typically remain intact and unaffected. Dead cells present in the sample prior to the matrix lysis can be removed prior to detection of the cells if necessary. Consequently, the method and the kit of the present invention offer a simple and fast way to isolate cells, preferably viable cells, from complex samples and - combined with sensitive detection methods like real time PCR - allow for fast and sensitive detection of pathogens in food and other complex samples.

The present invention is further illustrated by the following figures and examples, however, without being restricted thereto.

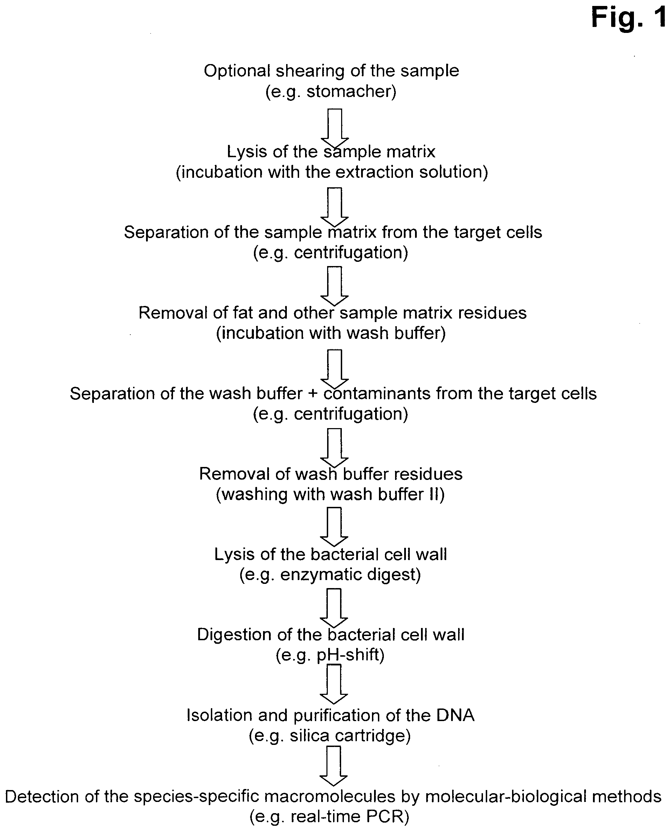

Fig. 1 gives one exemplary flow scheme for the procedural steps that have to be performed when using the method according to the present invention for detecting (qualitatively and/or quantitatively) pathogenic bacterial cells in complex samples like food samples.
Fig. 2 shows the results of the plate count quantification of *L. monocytogenes* and S. *Typhimurium* investigated in Application Example 5.

### Examples

The following examples represent practical applications of the invention.

### 1. Bacterial strains and culture conditions.

*Listeria monocytogenes* EGDe (1/2a, internal number 2964) is used as a model organism for Gram-positive bacteria and as a DNA quantification standard for real-time PCR. *Salmonella enterica* serovar Typhimurium (NCTC 12023) is used as a model organism for Gram-negative bacteria and as a DNA quantification standard for real-time PCR. The bacteria are maintained at -80°C using MicroBank™ technology (Pro-Lab Diagnostics, Richmont Hill, Canada) and are part of the collection of bacterial strains at the Institute of Milk Hygiene, Milk Technology and Food Science, University of Veterinary Medicine, Vienna, Austria. All bacterial strains are grown overnight in tryptone soya broth with 0.6% (w/v) yeast extract (TSB-Y; Oxoid, Hampshire, United Kingdom) at the respective optimal growth temperatures (37°C, *L. monocytogenes* and 42°C, S. Typhimurium).

### 2. Microscopic investigation.

Viability staining is performed by adding 1 µl of component A and 1 µl of component B of the Live/Dead^{®} *Bac*Light^{™} Bacterial Viability Kit (Molecular Probes, Willow Creek, OR, USA) to 1 ml of an appropriate dilution of the bacterial cultures in sterile filtered Ringer's solution (Merck, Darmstadt, Germany). The samples are incubated for 15 minutes (min) in the dark, 400 µl are filtered onto 0.22-µm-pore-sized 13-mm black polycarbonate filters (Millipore, Billerica, MA, USA) using a 5 ml syringe and a Swinnex filter holder (Millipore). 12.7 mm filter discs to test antibiotics (Schleicher & Schuell GmbH, Dassel, Germany) are placed beneath the polycarbonate filters in the filter holder for support. Fifteen fields per filter are analysed for each sample. The following formula is used to calculate the number of stained cells per ml sample: N = mean number of cells per field × (effective filtration area/area of the field) × (1/dilution factor) × (1/filtrated volume in ml). A Leitz Laborlux 8 fluorescence microscope (Leitz, Germany, Wetzlar) with a 470 nm filter and is used for microscopic analysis at one thousand-fold magnification.

### 3. Inoculation of foods.

For artificial contamination of food one millilitre of the overnight culture is transferred to one millilitre of fresh medium and incubated at the respective optimal growth temperature for three hours. Subsequently 100 µl of the appropriate dilutions in PBS (phosphate buffered saline) are added to the samples. The plate count method and tryptone soya agar plates supplemented with 0.6% (w/v) yeast extract (TSA-Y; Oxoid, Hampshire, United Kingdom) are used for quantification of all bacterial strains used. The agar plates are incubated at the respective optimal growth temperature for 24 hours. All sample matrices are purchased from local supermarkets. All samples used for artificial contamination are tested to be *L. monocytogenes* and S. Typhimurium negative, using the matrix lysis protocol and respective real-time PCR assays as described below. All inoculation experiments are performed in duplicate.

### 4 A. Matrix lysis with extraction solution comprising ionic liquids.

A 5% (v/v) aqueous solution of 1-ethyl-3-methylimidazolium thiocyanate ([emim]SCN; Merck KGaA, Darmstadt, Germany) is used for ice cream and egg. A 7.5% (v/v) aqueous solution of [emim]SCN is used for ultra high temperature (UHT) milk. If not otherwise indicated matrix lysis is performed as follows: 12.5 g of liquid or 6.25 g of solid foodstuff are mixed with 10 ml lysis buffer and homogenized twice each in the Stomacher 400 (Seward, London, UK) laboratory blender for 3 min each. The homogenate is transferred to 50 ml polypropylene tubes (Corning, NY, USA). Lysis buffer is added to bring the volume to 45 ml. The samples are incubated horizontally in a water bath (at 37°C for *L. monocytogenes* or 42°C for S. Typhimurium, respectively) and shaken at 200 rpm for 30 min. The samples then are centrifuged at 3,220 × g for 30 min at room temperature. The pellet is re-suspended in 40 ml washing buffer (1% Lutensol AO-07, and PBS) and incubated horizontally in a water bath, shaken at 200 rpm for 30 min at the temperatures used during the lysis step. Afterwards, the samples are centrifuged at 3,220 × g for 30 min at room temperature and the supernatant is gently discarded. The pellet is re-suspended in 500 µl PBS, transferred to a 1.5 ml plastic tube (Eppendorf, Hamburg, Germany) and washed twice in 1 ml PBS with additional centrifugation for 5 min at 5,000 × g.

### 4 B. Matrix lysis with extraction solution comprising MgCl₂.

The lysis buffer (= extraction solution) contains 0.5 to 3 M MgCl₂, 1 x Tris buffer, pH 5-7.

12 g of liquid or 6 g of solid foodstuff are mixed with 10 ml lysis buffer and homogenized twice each in the Stomacher 400 (Seward, London, UK) laboratory blender for 3 min each. The homogenate is transferred to 50 ml polypropylene tubes (Corning, NY, USA). Lysis buffer is added to bring the volume to 45 ml. The samples are incubated horizontally in a water bath (at 37°C for *L. monocytogenes* or 42°C for *S*. Typhimurium, respectively) and shaken at 200 rpm for 30 min. The samples then are centrifuged at 3,220 × g for 30 min at room temperature. The supernatant is carefully removed leaving about 500 µl of the sample in the tube. The remaining sample and pellet is re-suspended in 40 ml washing buffer (1% Lutensol AO-07, and 1 x PBS) and incubated horizontally in a water bath, shaken at 200 rpm for 30 min at the temperatures used during the lysis step. Afterwards, the samples are centrifuged at 3,220 × g for 30 min at room temperature and the supernatant is gently discarded to leave about 250 µl of the sample in the tube. The remaining sample and pellet is re-suspended in 500 µl 1 x PBS, transferred to a 1.5 ml plastic tube (Eppendorf, Hamburg, Germany). Afterwards, the samples are centrifuged for 5 min at 5,000 × g at room temperature and the supernatant is gently discarded. The remaining pellet is washed twice in 1 ml PBS with additional centrifugation for 5 min at 5,000 × g.

### 5. DNA isolation.

DNA isolation from the remaining bacterial pellet following matrix lysis is performed using the NucleoSpin^{®} tissue kit (Machery-Nagel, Düren, Germany) and the support protocol for Gram-positive bacteria. The final step of the protocol is modified and therefore two times 50 µl of double distilled water are used to elute the DNA from the column.

### 6. Viable cell quantification.

Viable cell quantification from the remaining bacterial pellet following matrix lysis is performed using the plate count method (PCM) on both, unselective tryptone soya agar plates supplemented with 0.6% (w/v) yeast extract (TSA-Y; Oxoid, Hampshire, United Kingdom). Selective xylose lysine deoxycholate agar (XLD; Oxoid, Hampshire, United Kingdom) is used for S. Typhimurium and Oxoid Chromogenic Listeria Agar (OCLA; Oxoid, Hampshire, United Kingdom) for *L. monocytogenes.*

### 7. DNA standard for real-time PCR quantification.

The genomic DNA of one millilitre overnight culture of *L. monocytogenes* is extracted by using the NucleoSpin^{®} tissue kit (Macherey - Nagel) and the support protocol for Gram-positive bacteria. DNA concentration is analytically determined by fluorimetric measurment using a Hoefer DyNA Quant200 apparatus (Pharmacia Biotech, San Francisco, CA, USA) and a 8452A Diode Array Spectrophotometer (Hewlett Packard, Palo Alto, CA, USA). The copy number of the *prfA* gene is determined by assuming that, based on the molecular weight of the genome of *L. monocytogenes*, 1 ng of DNA equals 3.1 × 10⁵ copies of the entire genome, and that the *prfA* gene is a single-copy gene. The copy numbers of the *Salmonella* target were similarly determined by assuming 1.9 × 10⁵ copies of the entire S. Typhimurium genome per 1 ng of DNA.

### 8. Real-time PCR.

Real-time PCR detection of *L. monocytogenes* by targeting a 274 bp fragment of the *prfA* gene is performed according to previously published formats (P. Rossmanith et al., Research in Microbiology, 157 (2006) 763-771)). S. Typhimurium is detected using the SureFood® Kit (R-Biofarm, Darmstadt, Germany), according to the instruction manual. Real-time PCR is performed in an Mx3000p real-time PCR thermocycler (Stratagene, La Jolla, CA, USA). The 25 µl volume containes 5 µl of DNA template. Real-time PCR results are expressed as bacterial cell equivalents (BCE). All real-time PCR reactions are performed in duplicate.

### Application Examples

### 1. Real-time PCR of S. Typhimurium from ice cream and eggs following matrix lysis.

Artificially contaminated ice cream and eggs, containing a 4-step decimal dilution series of S. Typhimurium starting at 6.67 × 10⁵ CFU (standard derivation (SD): ± 2.54 × 10⁵) per 6.25 g of sample, is subjected to DNA isolation and real-time PCR after matrix lysis. The average number of BCE per sample obtained by real-time PCR from ice cream is 3.31 × 10⁶ (SD: ± 4.00 × 10⁵) and 5.02 × 10⁵ (SD: ± 2.87 × 10⁵) from egg for 6.67 × 10⁵ CFU inoculated cells, 3.34 × 10⁵ (SD: ± 4.57 × 10⁴) and 9.23 × 10⁵ (SD: ± 6.26 × 10⁴) from egg for 6.67 × 10⁴ CFU inoculated cells, 2.68 × 10⁴ (SD: ± 4.73 × 10³) and 1.30 × 10⁴ (SD: ± 2.73 × 10³) from egg for 6.67 × 10³ CFU inoculated cells and 2.74 × 10³ (SD: ± 1.46 × 10³) and 8.11 × 10² (SD: ± 4.82 × 10²) from egg for 6.67 × 10² CFU inoculated cells (Table 1). The average number of BCE achieved for the DNA isolation efficiency control sample before matrix lysis is 3.06 × 10⁴ (SD: ± 3.06 × 10³) for 6.67 × 10³ CFU inoculated cells. The respective average amount of inoculated bacterial cells counted by means of microscopic cell counts is 1.84 × 10⁴ (SD: ± 4.97 × 10³) (Table 4).

### 2. Real-time PCR of L. monocytogenes from UHT milk following matrix lysis.

Artificially contaminated UHT milk, containing a 4-step decimal dilution series of *L. monocytogenes* starting at 1.14 × 10⁶ CFU (SD: ± 2.28 × 10⁵) per 12.5 ml of sample, is subjected to DNA isolation and real-time PCR after matrix lysis. The average number of BCE per sample obtained by real-time PCR from UHT milk is 1.70 × 10⁶ (SD: ± 1.90 × 10⁵) for 1.14 × 10⁶ CFU inoculated cells, 1.49 × 10⁵ (SD: ± 2.22 × 10⁴) for 1.14 × 10⁵ CFU inoculated cells, 1.60 × 10⁴ (SD: ± 3.27 × 10³) for 1.14 × 10⁴ CFU inoculated cells and 1.97 × 10³ (SD: ± 7.09 × 10²) for 1.14 × 10³ CFU inoculated cells (Table 1). The average number of BCE achieved for the DNA isolation efficiency control sample before matrix lysis is 1.48 × 10⁴ (SD: ± 1.93 × 10³) for 1.14 × 10⁴ CFU inoculated cells. The respective average amount of inoculated bacterial cells counted by means of microscopic cell counts is 2.94 × 10⁴ (SD: ± 7.64 × 10³) (Table 4).

According to the protocols given in application Example 1 and 2, the matrix lysis protocol using an extraction solution comprising at least one ionic liquid is tested in combination with real-time PCR to demonstrate the ability for direct quantification of *L. monocytogenes* from UHT milk, as well as of S. Typhimurium from ice cream and eggs. In comparison with the CFU of the inoculate before matrix lysis, bacterial cell equivalent (BCE) recovery rates of 190% for *L. monocytogenes* from 12.5 ml UHT milk and of 298% for *S*. Typhimurium from 6.25 g ice cream and eggs are obtained after matrix lysis (Table 4). These recovery rates are the result of an underestimation of the actual cell count per sample by applying the PCM. This conclusion is verified by the fact that the BCE counts after matrix lysis correlates much better with the cell counts of the microscopic investigation performed to count the inoculate before matrix lysis and the real-time PCR control results (Table 4). In comparison with the cell counts by microscopic investigation of the inoculate before matrix lysis, *L. monocytogenes* is recovered from milk with 75% and S. Typhimurium with 108%. In comparison with the real-time PCR control before matrix lysis, *L*. *monocytogenes* is recovered from milk with 114% and *S*. Typhimurium with 65% (Table 4). The recovery rates for *L. monocytogenes* and *S*. Typhimurium are consistent for all inoculation levels and all foodstuffs tested (Table 1). This demonstrated that the matrix lysis protocol using an extraction solution comprising at least one ionic liquid enables adequate contaminate differentiation in log scale measures.

**TABLE 1. Real-time PCR quantification of L. monocytogenes and S. Typhimurium from various foodstuffs after matrix lysis**

| | *L. monocytogenes* | *S.* Typhimurium | |
|---|---|---|---|
| | Inoculation level of the foodstuffs before matrix lysis CFU*^{a}*/ml (SD*^{b}*) | | |
| | 1.14 × 10⁹ (± 2.28 × 10⁸) | 6.67 × 10⁸ (± 2.54 × 10⁸) | |

| | Recovery after matrix lysis BCE*^{c}*/ml (SD) | | |
|---|---|---|---|
| Dil. rate*^{d}* × | milk (UHT) | egg | ice cream |
| 10⁻³ | 1.70 × 10⁶ (± 1.90 × 10⁵) | 5.02 × 10⁵ (± 2.87 × 10⁵) | 3.31 × 10⁶ (± 4.00 × 10⁵) |
| 10⁻⁴ | 1.49 × 10⁵ (± 2.22 × 10⁴) | 9.23 × 10⁴ (± 6.26 × 10⁴) | 3.34 × 10⁵ (± 4.57 × 10⁴) |
| 10⁻⁵ | 1.60 × 10⁴ (± 3.27 × 10³) | 1.30 × 10⁴ (± 2.73 × 10³) | 2.68 × 10⁴ (± 4.73 × 10³) |
| 10⁻⁶ | 1.97 × 10³ (± 7.09 × 10²) | 8.11 × 10² (± 4.82 × 10²) | 2.74 × 10³ (± 1.46 × 10³) |

| | | | |
|---|---|---|---|
| a CFU: colony forming units as obtained by plate count. b SD.: standard deviation c BCE.: bacterial cell equivalent (in terms of real-time PCR counts) d Dilution series from the initial inoculation level concentrations | | | |

### 3. Plate count quantification of L. monocytogenes and S. Typhimurium from foodstuffs following matrix lysis.

Artificially contaminated foodstuffs, containing a 4-step decimal dilution series of either *L. monocytogenes* or *S*. Typhimurium, are subjected to plate count quantification after matrix lysis. The average recovery of *L*. *monocytogenes* from 12.5 ml samples is 108% on TSA-Y agar plates in comparison with the control sample. *S*. Typhimurium is recovered from 6.25 g samples with an average 60% from eggs on TSA-Y agar plates. Quantification of *S*. Typhimurium on TSA-Y agar from ice cream is not possible because of the microbial background flora of the foodstuff. An average recovery of 36% is achieved (Table 2) when selective XLD agar is used.

On selective agar plates recovery rates are reduced in comparison with unselective agar plates. *L. monocytogenes* is quantified from 12.5 ml UHT milk with an average recovery of 68% on OCLA agar and S. Typhimurium with 34% from 6.25 g eggs on XLD agar, respectively (Table 3). These results correlate with the known fact that bacterial growth on selective agar plates may be reduced in comparison with growth on unselective agar plates.

The recovery rates for both organisms are consistent for all inoculation levels, which shows that the matrix lysis protocol enables proper contaminant differentiation in log scale measures. However, considering the high standard derivation and the observed underestimation of the actual cell counts (Table 4), the PCM seems to be less appropriate for quantification purposes in comparison with real-time PCR.

**TABLE 2. Viable cell quantification of L. monocytogenes and S. Typhimurium from various foodstuffs after matrix lysis**

| | L. monocytogenes | S. Typhimurium | |
|---|---|---|---|
| | UHT milk*^{a}* | Egg*^{a}* | Ice cream*^{b}* |
| Control*^{c}* CFU/ml (RSD*^{d}*) | 4.30 × 10⁸ (26.4%) | 6.85 × 10⁸ (18.5%) | 6.99 × 10⁸ (19%) |
| Foodstuff avg. after matrix lysis CFU/ml (RSD) | 4.65 × 10⁸ (36.8%) | 4.14 × 10⁸ (37.4%) | 2.54 × 10⁸ (22.2%) |
| Recovery rate (%)*^{e}* | 108% | 60% | 36% |

| | | | |
|---|---|---|---|
| a Results are based on values from tryptone soya agar + 0.6% (w/v) yeast extract. b Results are based on values from xylose lysine deoxycholate agar. c Inoculation level of the foodstuffs before matrix lysis. d RSD.: relative standard deviation. e Recovery is calculated on the basis of the CFU counts before and after matrix lysis. | | | |

### 4. Comparison of plate count quantification of L. monocytogenes from UHT milk and S. Typhimurium from eggs following matrix lysis on unselective and selective agar plates.

6.25 g of artificially contaminated eggs, containing a 4-step decimal dilution series of *S*. Typhimurium with 6.85 × 10⁸ CFU/ml (relative standard derivation (RSD): 18.5%) are subjected to plate count quantification on TSA-Y and XLD agar plates after matrix lysis. The average number of CFU per sample obtained by PCM from egg is 4.14 × 10⁸ (RSD: 37.4%) on TSA-Y agar plates and 2.33 × 10⁸ (RSD: 12.5%) on XLD agar plates. The recovery rate of *S*. Typhimurium from egg is 34% on selective and 60% on unselective agar (Table 3).

12.5 ml of artificially contaminated UHT milk, containing a 4-step decimal dilution series of *L. monocytogenes* with 4.30 × 10⁸ CFU/ml (RSD: 26.4%) are subjected to plate count quantification on TSA-Y and OCLA agar plates after matrix lysis. The average number of CFU per sample obtained by PCM from UHT milk is 4.65 × 10⁸ (RSD: 36.8%) on TSA-Y agar plates and 2.90 × 10⁸ (RSD: 37.9%) on OCLA agar plates. The recovery rate of *L*. *monocytogenes* from UHT milk is 67% on selective and 108% on unselective agar (Table 3).

**TABLE 3. Comparison of viable cell counts of L. monocylogenes and S. Typhimurium from UHT milk and eggs after matrix lysis on selective (XLD; OCLA) and unselective (TSA-Y) agar plates**

| | *L. monocytogenes^{a}* | | *S.* Typhimurium*^{b}* | |
|---|---|---|---|---|
| | TSA-Y*^{c}* | OCLA*^{c}* | TSA-Y*^{c}* | XLD*^{c}* |
| Control*^{d}* before matrix lysis CFU/ml (RSD*^{e}*) | 4.30 × 10⁸ (26.4%) | - | 6.85 × 10⁸ (18.5%) | - |
| Foodstuff^{*a*,*b*} avg. after matrix lysis CFU/ml (RSD) | 4.65 × 10⁸ (36.8%) | 2.90 × 10⁸ (37.9%) | 4.14 × 10⁸ (37.4%) | 2.33 × 10⁸ (12.5%) |
| Recovery rate (%)*^{f}* | 108% | 67% | 60% | 34% |

| | | | | |
|---|---|---|---|---|
| a Foodstuff applied to matrix lysis: egg. b Foodstuff applied to matrix lysis: UHT milk c TSA-Y: Tryptone soya agar + 0.6% (w/v) yeast extract; XLD: Xylose lysine deoxycholate agar; OCLA: Oxoid chromogenic *Listeria* agar. d Inoculation level of the foodstuffs before matrix lysis. e RSD.: relative standard deviation. f Recovery is calculated on the basis of the CFU counts before and after matrix lysis. | | | | |

**TABLE 4. Determination of the recovery rate of L. monocytogenes and S. typhimurium from various foodotuffs (e,f) after matrix lysis as determined by real-time PCR**

| Recovery related to | Real-time PCR | | Inoculation level*^{a}* | |
|---|---|---|---|---|
| | Control*^{b}* BCE*^{c}*/ml (SD*^{d}*) | Foodstuffs avg.*^{e,f}* Samples after matrix lysis BCE/ml (SD) | Microscopy*^{a}* cells/ml (SD) | Plate count method*^{a}* CFU/ml (SD) |
| *L. monocytogenes^{e}* | 1.48 × 10⁴ (± 1.93 × 10³) | 1.69 × 10⁴ (± 1.71 × 10²) | 2.94 × 10⁴ (± 7.64 × 10³) | 1.14 × 10⁴ (± 2.21 × 10³) |

| Recovery rate (%)*^{g}* | | | | |
|---|---|---|---|---|
| Microscopy | 50% | 75% | 100% | - |
| Plate count method | 130% | 190% | - | 100% |
| Real-time PCR | 100% | 114% | - | - |
| ***S.* Typhimurium*^{f}*** | 3.06 × 10⁴ (± 3.06 × 10³) | 1.97 × 10⁴ (± 1.58 × 10³) | 1.84 × 10⁴ (± 4.97 × 10³) | 6.67 × 10³ (± 2.54 × 10³) |
| Recovery rate (%) | | | | |
| Microscopy | 166% | 108% | 100% | - |
| Plate count method | 459% | 295% | - | 100% |
| Real-time PCR | 100% | 65% | - | - |

| | | | | |
|---|---|---|---|---|
| a Inoculation level of the foodstuffs before matrix lysis. b Bacterial culture directly processed with NucleoSpin® tissue kit, without matrix lysis as control for DNA isolation efficiency. c BCE.: bacterial cell equivalent (in terms of real-time PCR counts) d SD.: standard deviation e Foodstuff applied to matrix lysis: UHT milk f Foodstuffs applied to matrix lysis: Ice cream and egg. g Recovery is calculated on the basis of the counts and values displayed in the respective vertical rows and compared to the related value representing 100%. | | | | |

### 5. Plate count quantification of L. monocytogenes and S. Typhimurium.

The influence of different MgCl₂ concentrations on the viability of *Listeria monocytogenes* and *Salmonella* Typhimurium is investigated. The target organisms are incubated for 30 min with 3 different concentrations of MgCl₂ (1 M, 2 M and 3 M) and at 3 different temperatures (35°C, 38°C and 45°C) and the CFUs on TSA-Y agar plates after the treatment are compared with the control sample.

2.78 × 10⁹ CFU/ml (relative standard derivation (RSD): 23%) *Listeria monocytogenes* cells are subjected to different concentrations of MgCl₂ and at different temperatures. With an incubation temperature of 35°C the CFU/ml of *L. monocytogenes* are 3.86 × 10⁹ CFU/ml (RSD: 22%) with 1 M MgCl₂, 3.10 × 10⁹ CFU/ml (RSD: 23%) with 2 M MgCl₂ and 1.76 × 10⁹ CFU/ml (RSD: 21%) with 3 M MgCl₂. With an incubation temperature of 38°C the CFU/ml of *L. monocytogenes* are 4.06 × 10⁹ CFU/ml (RSD: 31%) with 1 M MgCl₂, 3.64 × 10⁹ CFU/ml (RSD: 21%) with 2 M MgCl₂ and 8.5 × 10⁸ CFU/ml (RSD: 34%) with 3 M MgCl₂. With an incubation temperature of 45°C the CFU/ml of *L. monocytogenes* are 4.39 × 10⁹ CFU/ml (RSD: 23%) with 1 M MgCl₂, 1.68 × 10⁹ CFU/ml (RSD: 14%) with 2 M MgCl₂ and 1.5 × 10⁸ CFU/ml (RSD: 15%) with 3 M MgCl₂.

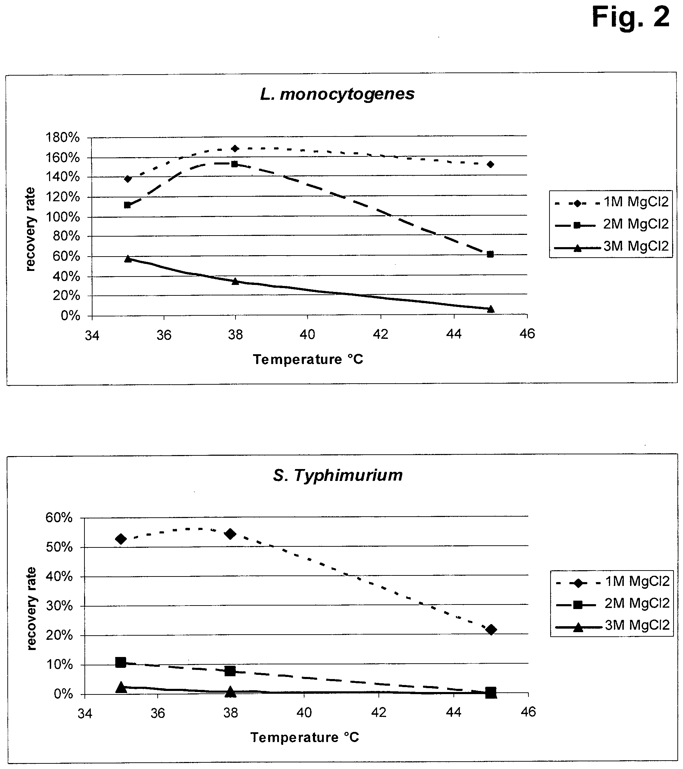

2.22 × 10⁹ CFU/ml (RSD: 18%) *Salmonella* Typhimurium cells are subjected to different concentrations of MgCl₂ and at different temperatures. With an incubation temperature of 35°C the CFU/ml of *S*. Typhimurium are 1.15 × 10⁹ CFU/ml (RSD: 37%) with 1 M MgCl₂, 2.3 × 10⁸ CFU/ml (RSD: 41%) with 2 M MgCl₂ and 5.75 × 10⁷ CFU/ml (RSD: 36%) with 3 M MgCl₂. With an incubation temperature of 38°C the CFU/ml of S. Typhimurium are 8.33 × 10⁸ CFU/ml (RSD: 22%) with 1 M MgCl₂, 1.35 × 10⁸ CFU/ml (RSD: 69%) with 2 M MgCl₂ and 2.0 × 10⁷ CFU/ml (RSD: 50%) with 3 M MgCl₂. With an incubation temperature of 45°C the CFU/ml of *S*. Typhimurium is 4.1 × 10⁸ CFU/ml (RSD: 23%) with 1 M MgCl₂. The results are visualized in Figure 2.

### 6. S. Typhimurium viable cell count of artificially contaminated ice cream after matrix lysis.

Artificially contaminated foodstuffs, containing a 4-step decimal dilution series of *S*. Typhimurium, are subjected to plate count quantification after matrix lysis. The matrix lysis protocol with 0.5 M MgCl₂ is tested on xylose lysine deoxycholate agar to demonstrate efficient direct quantification of *S*. Typhimurium from ice cream. *S*. Typhimurium is recovered from 6.5 g ice cream with an average of 38%.

## Claims

1. Method for isolating bacterial cells from a complex sample comprising the steps of:
a) providing a complex sample,
b) incubating said sample with an extraction solution that comprises MgCl₂ in concentrations between 0.5 and 3 M
c) isolating said cells from the mixture of step b) by centrifugation, affinity binding and/or filtration.

2. Method according to claim 1 **characterized in that** at least 30% of the cells isolated in step c) are viable cells.

3. Method according to claim 1 or 2 **characterized in that** the complex sample is a food sample.

4. Method according to one or more of claims 1 to 3, **characterized in that** the extraction solution comprises MgCl₂ in concentrations between 0.5 and 2 M.

5. Method according to one or more of claims 1 to 4, **characterized in that** the extraction solution does not comprise a detergent.

6. Method according to one or more of claims 1 to 5, **characterized in that** the sample is spiked with a defined amount of control cells prior to step b).

7. Method according to one or more of claims 1 to 6, **characterized in that** the sample is pre-incubated with a compound exhibiting osmotic stress-protective properties to the cells.

8. Method according to one or more of claims 1 to 7, **characterized in that** the sample is further incubated with at least one biopolymer degrading enzyme.

9. Method according to one or more of claims 1 to 8, **characterized in that** in a further step d) the cells are analyzed by cell counting, PCR methods, by using lectins or by methods involving antibodies, antimicrobial peptides (AMP), aptameres or viral binding domains, directed to surface structures of said cells.

## Patentansprüche

1. Verfahren zur Isolation von Bakterienzellen aus einer komplexen Probe, umfassend die Schritte:
a) Bereitstellen einer komplexen Probe,
b) Inkubieren der Probe mit einer Extraktionslösung, die MgCl₂ in Konzentrationen zwischen 0,5 und 3 M enthält,
c) Isolieren der Zellen aus dem Gemisch von Schritt b) durch Zentrifugation, Affinitätsbindung und/oder Filtration.

2. Verfahren nach Anspruch 1, **dadurch gekennzeichnet, dass** mindestens 30% der in Schritt c) isolierten Zellen lebende Zellen sind.

3. Verfahren nach Anspruch 1 oder 2, **dadurch gekennzeichnet, dass** die komplexe Probe eine Lebensmittelprobe ist.

4. Verfahren nach einem oder mehreren der Ansprüche 1 bis 3, **dadurch gekennzeichnet, dass** die Extraktionslösung MgCl₂ in Konzentrationen zwischen 0,5 und 2 M enthält.

5. Verfahren nach einem oder mehreren der Ansprüche 1 bis 4, **dadurch gekennzeichnet, dass** die Extraktionslösung kein Detergenz enthält.

6. Verfahren nach einem oder mehreren der Ansprüche 1 bis 5, **dadurch gekennzeichnet, dass** die Probe vor Schritt b) mit einer definierten Menge Kontrollzellen aufgestockt wird.

7. Verfahren nach einem oder mehreren der Ansprüche 1 bis 6, **dadurch gekennzeichnet, dass** die Probe mit einer Verbindung vorinkubiert wird, die den Zellen schützende Eigenschaften gegenüber osmotischem Stress verleiht.

8. Verfahren nach einem oder mehreren der Ansprüche 1 bis 7, **dadurch gekennzeichnet, dass** die Probe zusätzlich mit mindestens einem Biopolymer-abbauenden Enzym inkubiert wird.

9. Verfahren nach einem oder mehreren der Ansprüche 1 bis 8, **dadurch gekennzeichnet, dass** in einem weiteren Schritt d) die Zellen durch Zellzählung, PCR-Verfahren, durch Verwendung von Lectinen oder durch Verfahren mit Antikörpern, antimikrobiellen Peptiden (AMP), Aptameren oder viralen Bindungsdomänen, die gegen Oberflächenstrukturen der Zellen gerichtet sind, analysiert werden.

## Revendications

1. Méthode d'isolement de cellules bactériennes à partir d'un échantillon complexe, comprenant les étapes consistant à :
a) fournir un échantillon complexe,
b) incuber ledit échantillon avec une solution d'extraction comprenant du MgCl2 selon des concentrations comprises entre 0,5 et 3M,
c) isoler lesdites cellules du mélange de l'étape b) par centrifugation, fixation par affinité et/ou filtration.

2. Méthode selon la revendication 1, **caractérisée en ce qu'**au moins 30% des cellules isolées dans l'étape c) sont des cellules viables.

3. Méthode selon la revendication 1 à 2, **caractérisée en ce que** l'échantillon complexe est un échantillon d'aliment.

4. Méthode selon l'une ou plusieurs parmi les revendications 1 à 3, **caractérisée en ce que** la solution d'extraction comprend du MgCl₂ selon des concentrations comprises entre 0,5 et 2M.

5. Méthode selon l'une ou plusieurs parmi les revendications 1 à 4, **caractérisée en ce que** la solution d'extraction ne comprend pas de détergent.

6. Méthode selon l'une ou plusieurs parmi les revendications 1 à 5, **caractérisée en ce que** l'échantillon est dopé par une quantité définie de cellules témoins préalablement à l'étape b)

7. Méthode selon l'une ou plusieurs parmi les revendications 1 à 6, **caractérisée en ce que** l'échantillon est préincubé avec un composé conférant aux cellules des propriétés de protection vis-à-vis d'un stress osmotique.

8. Méthode selon l'une ou plusieurs parmi les revendications 1 à 7, **caractérisée en ce que** l'échantillon est incubé en outre avec au moins une enzyme de dégradation de biopolymères.

9. Méthode selon l'une ou plusieurs parmi les revendications 1 à 8, **caractérisée en ce que** dans une étape supplémentaire d), les cellules sont analysées par numération cellulaire, des méthodes de PCR, en utilisant des lectines ou par des méthodes impliquant des anticorps, des peptides anti-microbiens (AMP), des aptamères ou des domaines de fixation viraux, dirigés contre des structures de surface desdites cellules.
